# EUROPEAN PATENT SPECIFICATION

(11) **EP 4 311 522 B1**
(45) Date of publication and mention of the grant of the patent: **25.02.2026**
(21) Application number: 23185864.8
(22) Date of filing: 17.07.2023
(51) Int. Cl.: A61F 2/24

(54) **STRETCHABLE MEMBER TRANSCATHETER VALVE**
TRANSKATHETERVENTIL MIT DEHNBAREM ELEMENT
VALVE TRANSCATHÉTER À ÉLÉMENT ÉTIRABLE

(30) Priority: 26.07.2022 US 202263369458 P
(43) Date of publication of application: 31.01.2024
(73) Proprietor: St. Jude Medical, Cardiology Division, Inc., St. Paul MN 55117 (US)
(72) Inventor: PECKELS, William H., Robbinsdale, MN 55422 (US); MARNACH, Heath, Minneapolis, MN 55419 (US); HUDDLESTON, Preston James, Maplewood, MN 55117 (US); EIDENSCHINK, Tracee, Wayzata, MN 55391 (US)
(74) Representative: LKGLOBAL Partnerschaftsgesellschaft mbB

(56) References cited:
- US-A1- 2020 276 014
- US-A1- 2021 000 593
- US-A1- 2021 307 905
- US-A1- 2022 183 824

## Description

### Background of the Disclosure

Valvular heart disease, and specifically aortic and mitral valve disease, is a significant health issue in the United States. Valve replacement is one option for treating heart valve diseases. Prosthetic heart valves, including surgical heart valves and collapsible/expandable heart valves intended for transcatheter aortic valve replacement ("TAVR"), transcatheter mitral valve replacement ("TMVR") or transcatheter tricuspid valve replacement, are well known in the patent literature. Surgical or mechanical heart valves may be sutured into a native annulus of a patient during an open-heart surgical procedure, for example. Collapsible/expandable heart valves may be delivered into a patient via a tube-like delivery apparatus such as a catheter, a trocar, a laparoscopic instrument, or the like to avoid a more invasive procedure such as full open-chest, open-heart surgery. As used herein, reference to a "collapsible/expandable" heart valve includes heart valves that are formed with a small cross-section that enables them to be delivered into a patient through a tube-like delivery apparatus in a minimally invasive procedure, and then expanded to an operable state once in place, as well as heart valves that, after construction, are first collapsed to a small cross-section for delivery into a patient and then expanded to an operable size once in place in the valve annulus.

Collapsible/expandable prosthetic heart valves typically take the form of a one-way valve structure (often referred to herein as a valve assembly) mounted to/within an expandable stent. In general, these collapsible/expandable heart valves include a self-expanding or balloon-expandable stent, often made of nitinol or another shape-memory metal or metal alloy (for self-expanding stents) or steel or cobalt chromium (for balloon-expandable stents). Existing collapsible/expandable TAVR devices have been known to use different configurations of stent layouts - including straight vertical struts connected by "V"s as illustrated in U.S. Pat. No. 8,454,685, or diamond-shaped cell layouts as illustrated in U.S. Pat. No. 9,326,856. The one-way valve assembly mounted to/within the stent includes one or more leaflets, and may also include a cuff or skirt. The cuff may be disposed on the stent's interior or luminal surface, its exterior or abluminal surface, and/or on both surfaces. A cuff helps to ensure that blood does not just flow around the valve leaflets if the valve or valve assembly are not optimally seated in a valve annulus. A cuff, or a portion of a cuff disposed on the exterior of the stent, can help retard leakage around the outside of the valve (the latter known as paravalvular or "PV" leakage).

Balloon expandable valves are typically delivered to the native annulus while collapsed (or "crimped") onto a deflated balloon of a balloon catheter, with the collapsed valve being either covered or uncovered by an overlying sheath. Once the crimped prosthetic heart valve is positioned within the annulus of the native heart valve that is being replaced, the balloon is inflated to force the balloon expandable valve to transition from the collapsed or crimped condition into an expanded or deployed condition, with the prosthetic heart valve tending to remain in the shape into which it is expanded by the balloon. Typically, when the position of the collapsed prosthetic heart valve is determined to be in the desired position relative to the native annulus (e.g. via visualization under fluoroscopy), a fluid (typically a liquid although gas could be used as well) such as saline is pushed via a syringe (manually, automatically, or semi-automatically) through the balloon catheter to cause the balloon to begin to fill and expand, and thus cause the overlying prosthetic heart valve to expand into the native annulus. Alternatively, a prosthetic heart valve may be deployed within or adjacent a dock or other supporting structure.

When self-expandable prosthetic heart valves are delivered into a patient to replace a malfunctioning native heart valve, the self-expandable prosthetic heart valve is almost always maintained in the collapsed condition within a capsule of the delivery device. While the capsule may ensure that the prosthetic heart valve does not self-expand prematurely, the overlying capsule (with or without the help of additional internal retaining features) helps ensure that the prosthetic heart valve does not come into contact with any tissue prematurely, as well as helping to make sure that the prosthetic heart valve stays in the desired position and orientation relative to the delivery device during delivery.

US 2020/0276014 A1 describes an implantable device including a leaflet frame subcomponent and an anchor frame subcomponent that are configured to be delivered in a series configuration and subsequently nested or telescoped in-situ.

US 2022/0183824 A1 describes a valve prosthesis and a system for delivering a valve prosthesis, the system including a support frame, a valve anchor comprising an anchor leg and a plurality of U-shaped members, and a connection member coupled to the support frame and slidably coupled to the anchoring leg.

US 2021/0000593 A1 describes a stent and a replacement heart valve prosthesis with fixation features. US 2021/0307905 A1 describes a transcatheter delivery system.

It is a challenge to produce a system that includes a prosthetic heart valve and an accompanying delivery device with a diameter that small enough for successfully delivery. Specifically, conventional valves often stack the functional valve elements and the native anatomy engagement members, which results in an undesirably large diameter, even when crimpled within a delivery device.

### Brief Summary of the Disclosure

The above-mentioned challenge is addressed by the subject-matter of claim 1. Further embodiments are described in the dependent claims. The invention is set out in the appended claims and is described with reference to particular embodiments being illustrative of the principles and applications of the present invention.

In some embodiments, a prosthetic heart valve system includes an outer stent, an inner stent coupled to the outer stent, the inner stent supporting a valve assembly including a cuff and a plurality of leaflets, and at least one linking member coupling the outer stent to the inner stent, the at least one linking member being transitionable between a collapsed condition and a relaxed condition, the inner stent being axially spaced away from the outer stent when the at least one linking member is in the collapsed condition, and the inner stent being at least partially nested within the outer stent when the at least one linking member is in the relaxed condition.

### Brief Description of the Drawings

Fig. 1A is a perspective view of a stent of a prosthetic heart valve according to an embodiment of the disclosure.
Fig. 1B is a schematic front view of a section of the stent of Fig. 1A.
Fig. 1C is a schematic front view of a section of a stent according to an alternate embodiment of the prosthetic heart valve of Fig. 1A.
Figs. 1D-E are front views of the stent section of Fig. 1C in a collapsed and expanded state, respectively.
Figs. 1F-G are side views of a portion of the stent according to the embodiment of Fig. 1C in a collapsed and expanded state, respectively.
Fig. 1H is a flattened view of the stent according to the embodiment of Fig. 1C, as if cut and rolled flat.
Figs. 1I-J are front and side views, respectively, of a prosthetic heart valve including the stent of Fig. 1C.
Fig. 1K illustrates the view of Fig. 1H with an additional outer cuff provided on the stent.
Figs. 2A-B illustrates a prosthetic heart valve PHV, crimped over a balloon in the deflated and inflated conditions.
Fig. 3A is a schematic plan view of a prosthetic heart valve PHV according to one embodiment of the disclosure.
Figs. 3B-E are schematic side views showing the prosthetic heart valve PHV of Fig. 3A within a delivery device and at various stages of expansion, and a cross-sectional view of the inner stent disposed within the outer stent when delivered.
Figs. 4A-C are a schematic side view of another embodiment of a prosthetic heart valve PHV within a delivery device, a top view of the prosthetic heart valve after expansion, and cross-sectional view of the inner stent disposed within the outer stent when delivered.
Figs. 5A-B are a schematic perspective views of another embodiment of a prosthetic heart valve PHV before and after nesting.
Figs. 6A-B are schematic perspective views of a spiral inner connection of a prosthetic heart valve PHV during the collapsed and expanded conditions.

### Detailed Description

As used herein, the term "inflow end" when used in connection with a prosthetic heart valve refers to the end of the prosthetic valve into which blood first enters when the prosthetic valve is implanted in an intended position and orientation, while the term "outflow end" refers to the end of the prosthetic valve where blood exits when the prosthetic valve is implanted in the intended position and orientation. Thus, for a prosthetic aortic valve, the inflow end is the end nearer the left ventricle while the outflow end is the end nearer the aorta. The intended position and orientation are used for the convenience of describing the valve disclosed herein, however, it should be noted that the use of the valve is not limited to the intended position and orientation, but may be deployed in any type of lumen or passageway. For example, although the prosthetic heart valve is described herein as a prosthetic aortic valve, the same or similar structures and features can be employed in other heart valves, such as the pulmonary valve, the mitral valve, or the tricuspid valve. Further, the term "proximal," when used in connection with a delivery device or system, refers to a direction relatively close to the user of that device or system when being used as intended, while the term "distal" refers to a direction relatively far from the user of the device. In other words, the leading end of a delivery device or system is positioned distal to a trailing end of the delivery device or system, when being used as intended. As used herein, the terms "substantially," "generally," "approximately," and "about" are intended to mean that slight deviations from absolute are included within the scope of the term so modified. As used herein, the stent may assume an "expanded state" and a "collapsed state," which refer to the relative radial size of the stent.

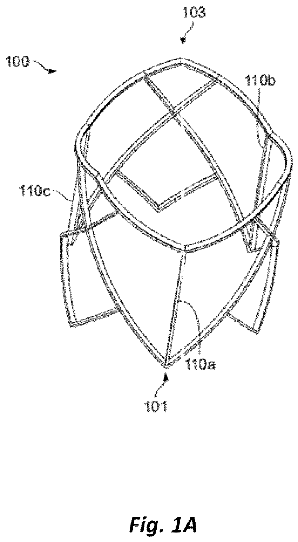

Fig. 1A illustrates a perspective view of a stent 100 of a prosthetic heart valve according to an embodiment of the disclosure. Stent 100 may include a frame extending in an axial direction between an inflow end 101 and an outflow end 103. Stent 100 includes three generally symmetric sections, wherein each section spans about 120 degrees around the circumference of stent 100. Stent 100 includes three vertical struts 110a, 110b, 110c, that extend in an axial direction substantially parallel to the direction of blood flow through the stent, which may also be referred to as a central longitudinal axis. Each vertical strut 110a, 110b, 110c may extend substantially the entire axial length between the inflow end 101 and the outflow end 103 of the stent 100, and may be disposed between and shared by two sections. In other words, each section is defined by the portion of stent 100 between two vertical struts. Thus, each vertical strut 110a, 110b, 110c is also separated by about 120 degrees around the circumference of stent 100. It should be understood that, if stent 100 is used in a prosthetic heart valve having three leaflets, the stent may include three sections as illustrated. However, in other embodiments, if the prosthetic heart valve has two leaflets, the stent may only include two of the sections.

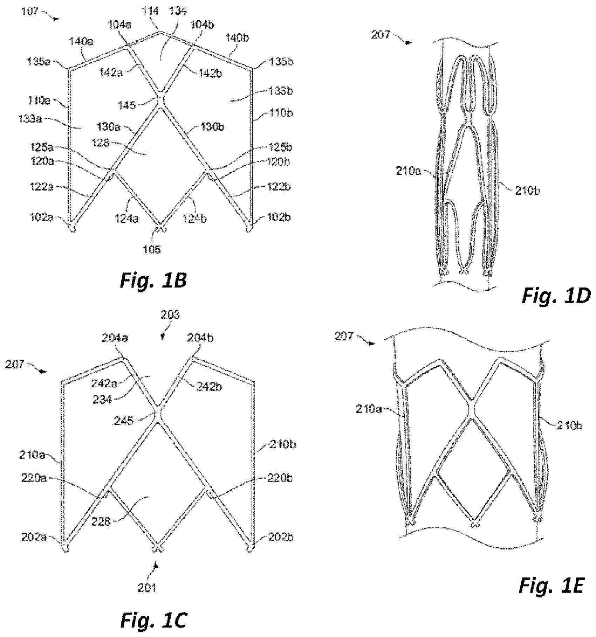

Fig. 1B illustrates a schematic view of a stent section 107 of stent 100, which will be described herein in greater detail and which is representative of all three sections. Stent section 107 depicted in Fig. 1B includes a first vertical strut 110a and a second vertical strut 110b. First vertical strut 110a extends axially between a first inflow node 102a and a first outer node 135a. Second vertical strut 110b extends axially between a second inflow node 102b and a second outer node 135b. As is illustrated, the vertical struts 110a, 110b may extend almost the entire axial length of stent 100. In some embodiments, stent 100 may be formed as an integral unit, for example by laser cutting the stent from a tube. The term "node" may refer to where two or more struts of the stent 100 meet one another. A pair of sequential inverted V's extends between inflow nodes 102a, 102b, which includes a first inflow inverted V 120a and a second inflow inverted V 120b coupled to each other at an inflow node 105. First inflow inverted V 120a comprises a first outer lower strut 122a extending between first inflow node 102a and a first central node 125a. First inflow inverted V 120a further comprises a first inner lower strut 124a extending between first central node 125a and inflow node 105. A second inflow inverted V 120b comprises a second inner lower strut 124b extending between inflow node 105 and a second central node 125b. Second inflow inverted V 120b further comprises a second outer lower strut 122b extending between second central node 125b and second inflow node 102b. Although described as inverted V's, these structures may also be described as half-cells, each half cell being a half-diamond cell with the open portion of the half-cell at the inflow end 101 of the stent 100.

Stent section 107 further includes a first central strut 130a extending between first central node 125a and an upper node 145. Stent section 107 also includes a second central strut 130b extending between second central node 125b and upper node 145. First central strut 130a, second central strut 130b, first inner lower strut 124a and second inner lower strut 124b form a diamond cell 128. Stent section 107 includes a first outer upper strut 140a extending between first outer node 135 and a first outflow node 104a. Stent section 107 further includes a second outer upper strut 140b extending between second outer node 135b and a second outflow node 104b. Stent section 107 includes a first inner upper strut 142a extending between first outflow node 104a and upper node 145. Stent section 107 further includes a second inner upper strut 142b extending between upper node 145 and second outflow node 104b. Stent section 107 includes an outflow inverted V 114 which extends between first and second outflow nodes 104a, 104b. First vertical strut 110a, first outer upper strut 140a, first inner upper strut 142a, first central strut 130a and first outer lower strut 122a form a first generally kite-shaped cell 133a. Second vertical strut 110b, second outer upper strut 140b, second inner upper strut 142b, second central strut 130b and second outer lower strut 122b form a second generally kite-shaped cell 133b. First and second kite-shaped cells 133a, 133b are symmetric and opposite each other on stent section 107. Although the term "kite-shaped," is used above, it should be understood that such a shape is not limited to the exact geometric definition of kite-shaped. Outflow inverted V 114, first inner upper strut 142a and second inner upper strut 142b form upper cell 134. Upper cell 134 is generally kite-shaped and axially aligned with diamond cell 128 on stent section 107. It should be understood that, although designated as separate struts, the various struts described herein may be part of a single unitary structure as noted above. However, in other embodiments, stent 100 need not be formed as an integral structure and thus the struts may be different structures (or parts of different structures) that are coupled together.

Fig. 1C illustrates a schematic view of a stent section 207 according to an alternate embodiment of the disclosure. Unless otherwise stated, like reference numerals refer to like elements of above-described stent 100 but within the 200-series of numbers. Stent section 207 is substantially similar to stent section 107, including inflow nodes 202a, 202b, vertical struts 210a, 210b, first and second inflow inverted V's 220a, 220b and outflow nodes 204a, 204b. The structure of stent section 207 departs from that of stent section 107 in that it does not include an outflow inverted V. The purpose of an embodiment having such structure of stent section 207 shown in Fig. 1C is to reduce the required force to expand the outflow end 203 of the stent 200, compared to stent 100, to promote uniform expansion relative to the inflow end 201. Outflow nodes 204a, 204b are connected by a properly oriented V formed by first inner upper strut 242a, upper node 245 and second inner upper strut 242b. In other words, struts 242a, 242b may form a half diamond cell 234, with the open end of the half-cell oriented toward the outflow end 203. Half diamond cell 234 is axially aligned with diamond cell 228. Adding an outflow inverted V coupled between outflow nodes 204a, 204b contributes additional material that increases resistance to modifying the stent shape and requires additional force to expand the stent. The exclusion of material from outflow end 203 decreases resistance to expansion on outflow end 203, which may promote uniform expansion of inflow end 201 and outflow end 203. In other words, the inflow end 201 of stent 200 does not include continuous circumferential structure, but rather has mostly or entirely open half-cells with the open portion of the half-cells oriented toward the inflow end 201, whereas most of the outflow end 203 includes substantially continuous circumferential structure, via struts that correspond with struts 140a, 140b. All else being equal, a substantially continuous circumferential structure may require more force to expand compared to a similar but open structure. Thus, the inflow end 101 of stent 100 may require more force to radially expand compared to the outflow end 103. By omitting inverted V 114, resulting in stent 200, the force required to expand the outflow end 203 of stent 200 may be reduced to an amount closer to the inflow end 201.

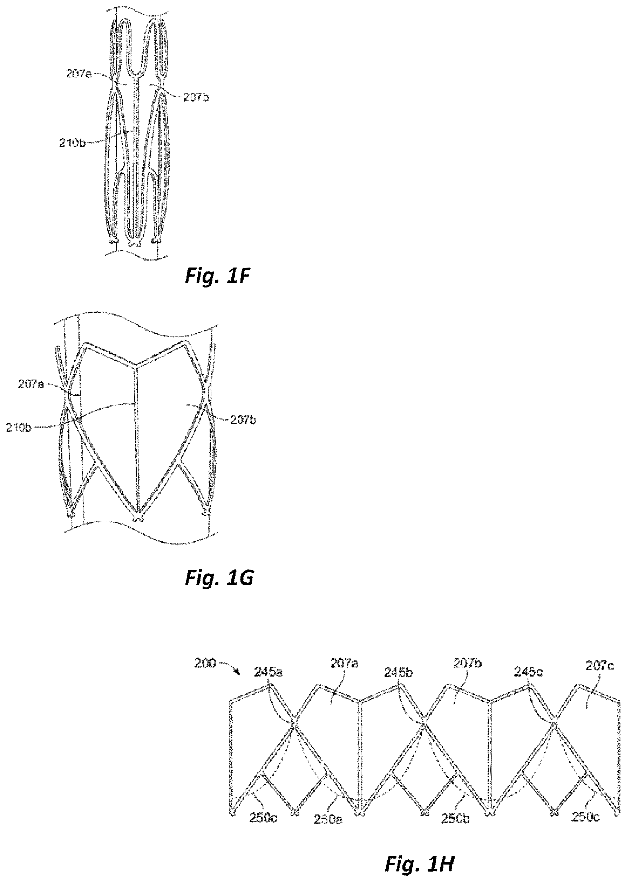

Fig. 1D shows a front view of stent section 207 in a collapsed state and Fig. 1E shows a front view of stent section 207 in an expanded state. It should be understood that stent 200 in Figs. 1D-E is illustrated with an opaque tube extending through the interior of the stent, purely for the purpose of helping illustrate the stent, and which may represent a balloon over which the stent section 207 is crimped. As described above, a stent comprises three symmetric sections, each section spanning about 120 degrees around the circumference of the stent. Stent section 207 illustrated in Figs. 1D-E is defined by the region between vertical struts 210a, 210b. Stent section 207 is representative of all three sections of the stent. Stent section 207 has an arcuate structure such that when three sections are connected, they form one complete cylindrical shape. Figs. 1F-G illustrate a portion of the stent from a side view. In other words, the view of stent 200 in Figs. 1F-G is rotated about 60 degrees compared to the view of Figs. 1D-E. The view of the stent depicted in FIGS. 1F-G is centered on vertical strut 210b showing approximately half of each of two adjacent stent sections 207a, 207b on each side of vertical strut 210b. Sections 207a, 207b surrounding vertical strut 210b are mirror images of each other. Fig. 1F shows stent sections 207a, 207b in a collapsed state whereas Fig. 1G shows stent sections 207a, 207b in an expanded state.

Fig. 1H illustrates a flattened view of stent 200 including three stent sections 207a, 207b, 207c, as if the stent has been cut longitudinally and laid flat on a table. As depicted, sections 207a, 207b, 207c are symmetric to each other and adjacent sections share a common vertical strut. As described above, stent 200 is shown in a flattened view, but each section 207a, 207b, 207c has an arcuate shape spanning 120 degrees to form a full cylinder. Further depicted in Fig. 1H are leaflets 250a, 250b, 250c coupled to stent 200. However, it should be understood that only the connection of leaflets 250a-c is illustrated in Fig. 1H. In other words, each leaflet 250a-c would typically include a free edge, with the free edges acting to coapt with one another to prevent retrograde flow of blood through the stent 200, and the free edges moving radially outward toward the interior surface of the stent to allow antegrade flow of blood through the stent. Those free edges are not illustrated in Fig. 1H. Rather, the attached edges of the leaflets 250a-c are illustrated in dashed lines in Fig. 1H. Although the attachment may be via any suitable modality, the attached edges may be preferably sutured to the stent 200 and/or to an intervening cuff or skirt between the stent and the leaflets 250a-c. Each of the three leaflets 250a, 250b, 250c, extends about 120 degrees around stent 200 from end to end and each leaflet includes a belly that may extend toward the radial center of stent 200 when the leaflets are coapted together. Each leaflet extends between the upper nodes of adjacent sections. First leaflet 250a extends from first upper node 245a of first stent section 207a to second upper node 245b of second stent section 207b. Second leaflet 250b extends from second upper node 245b to third upper node 245c of third stent section 207c. Third leaflet 250c extends from third upper node 245c to first upper node 245a. As such, each upper node includes a first end of a first leaflet and a second end of a second leaflet coupled thereto. In the illustrated embodiment, each end of each leaflet is coupled to its respective node by suture. However, any coupling means may be used to attach the leaflets to the stent. It is further contemplated that the stent may include any number of sections and/or leaflets. For example, the stent may include two sections, wherein each section extends 180 degrees around the circumference of the stent. Further, the stent may include two leaflets to mimic a bicuspid valve. Further, it should be noted that each leaflet may include tabs or other structures (not illustrated) at the junction between the free edges and attached edges of the leaflets, and each tab of each leaflet may be coupled to a tab of an adjacent leaflet to form commissures. In the illustrated embodiment, the leaflet commissures are illustrated attached to nodes where struts intersect. However, in other embodiments, the stent 200 may include commissure attachment features built into the stent to facilitate such attachment. For example, commissures attachment features may be formed into the stent 200 at nodes 245a-c, with the commissure attachment features including one or more apertures to facilitate suturing the leaflet commissures to the stent. Further, leaflets 250a-c may be formed of a biological material, such as animal pericardium, or may otherwise be formed of synthetic materials, such as ultra-high molecular weight polyethylene (UHMWPE).

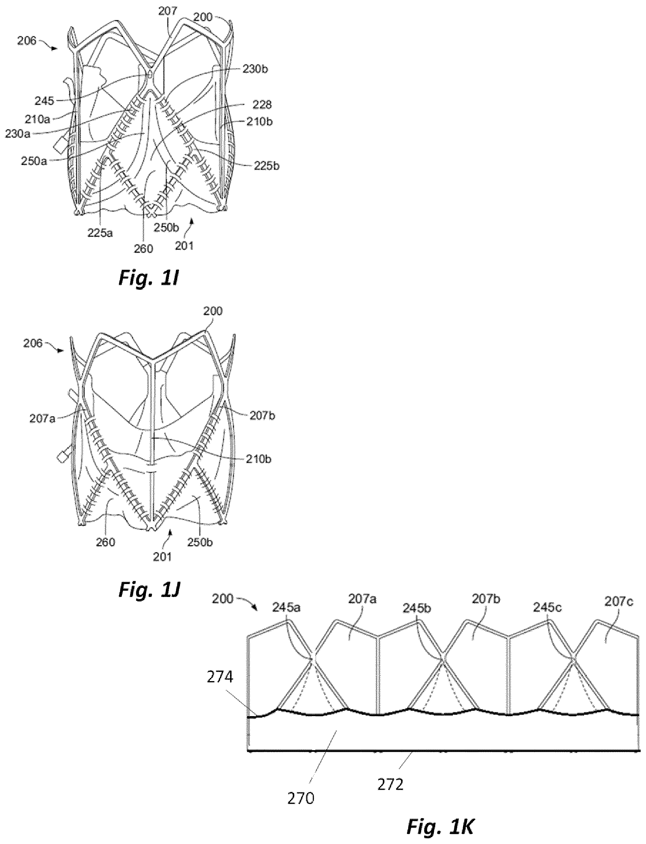

Figs. 1I-J illustrate prosthetic heart valve 206, which includes stent 200, a cuff 260 coupled to stent 200 (for example via sutures) and leaflets 250a, 250b, 250c attached to stent 200 and/or cuff 260 (for example via sutures). Prosthetic heart valve 206 is intended for use in replacing an aortic valve, although the same or similar structures may be used in a prosthetic valve for replacing other heart valves. Cuff 260 is disposed on a luminal or interior surface of stent 200, although the cuff could be disposed alternately or additionally on an abluminal or exterior surface of the stent. The cuff 260 may include an inflow end disposed substantially along inflow end 201 of stent 200. Fig. 1I shows a front view of valve 206 showing one stent portion 207 between vertical struts 210a, 210b including cuff 260 and an outline of two leaflets 250a, 250b sutured to cuff 260. Different methods of suturing leaflets to the cuff as well as the leaflets and/or cuff to the stent may be used, many of which are described in U.S. Pat. No. 9,326,856. In the illustrated embodiment, the upper (or outflow) edge of cuff 260 is sutured to first central node 225a, upper node 245 and second central node 225b, extending along first central strut 230a and second central strut 230b. The upper (or outflow) edge of cuff 260 continues extending approximately between the second central node of one section and the first central node of an adjacent section. Cuff 260 extends between upper node 245 and inflow end 201. Thus, cuff 260 covers the cells of stent portion 207 formed by the struts between upper node 245 and inflow end 201, including diamond cell 228. Fig. 1J illustrates a side view of stent 200 including cuff 260 and an outline of leaflet 250b. In other words, the view of valve 206 in Fig. 1J is rotated about 60 degrees compared to the view of Fig.1I. The view depicted in Fig. 1J is centered on vertical strut 210b showing approximately half of each of two adjacent stent sections 207a, 207b on each side of vertical strut 210b. Sections 207a, 207b surrounding vertical strut 210b are mirror images of each other. As described above, the cuff may be disposed on the stent's interior or luminal surface, its exterior or abluminal surface, and/or on both surfaces. A cuff ensures that blood does not just flow around the valve leaflets if the valve or valve assembly are not optimally seated in a valve annulus. A cuff, or a portion of a cuff disposed on the exterior of the stent, can help retard leakage around the outside of the valve (the latter known as paravalvular leakage or "PV" leakage). In the embodiment illustrated in Figs. 1I-J, the cuff 260 only covers about half of the stent 200, leaving about half of the stent uncovered by the cuff. With this configuration, less cuff material is required compared to a cuff that covers more or all of the stent 200. Less cuff material may allow for the prosthetic heart valve 206 to crimp down to a smaller profile when collapsed. It is contemplated that the cuff may cover any amount of surface area of the cylinder formed by the stent. For example, the upper edge of the cuff may extend straight around the circumference of any cross section of the cylinder formed by the stent. Cuff 260 may be formed of any suitable material, including a biological material such as animal pericardium, or a synthetic material such as UHMWPE.

As noted above, Figs. 1I-Jillustrate a cuff 260 positioned on an interior of the stent 200. An example of an additional outer cuff 270 is illustrated in Fig. 1K. It should be understood that outer cuff 270 may take other shapes than that shown in Fig. 1K. The outer cuff 270 shown in Fig. 1K may be included without an inner cuff 260, but preferably is provided in addition to an inner cuff 260. The outer cuff 270 may be formed integrally with the inner cuff 260 and folded over (e.g., wrapped around) the inflow edge of the stent, or may be provided as a member that is separate from inner cuff 260. Outer cuff 270 may be formed of any of the materials described herein in connection with inner cuff 260. In the illustrated embodiment, outer cuff 270 includes an inflow edge 272 and an outflow edge 274. If the inner cuff 260 and outer cuff 270 are formed separately, the inflow edge 272 may be coupled to an inflow end of the stent 200 and/or an inflow edge of the inner cuff 260, for example via suturing, ultrasonic welding, or any other suitable attachment modality. The coupling between the inflow edge 272 of the outer cuff 270 and the stent 200 and/or inner cuff 260 preferably results in a seal between the inner cuff 260 and outer cuff 270 at the inflow end of the prosthetic heart valve so that any retrograde blood that flows into the space between the inner cuff 260 and outer cuff 270 is unable to pass beyond the inflow edges of the inner cuff 260 and outer cuff 270. The outflow edge 274 may be coupled at selected locations around the circumference of the stent 200 to struts of the stent 200 and/or to the inner cuff 260, for example via sutures. With this configuration, an opening may be formed between the inner cuff 260 and outer cuff 270 circumferentially between adjacent connection points, so that retrograde blood flow will tend to flow into the space between the inner cuff 260 and outer cuff 270 via the openings, without being able to continue passing beyond the inflow edges of the cuffs. As blood flows into the space between the inner cuff 260 and outer cuff 270, the outer cuff 270 may billow outwardly, creating even better sealing between the outer cuff 270 and the native valve annulus against which the outer cuff 270 presses. The outer cuff 270 may be provided as a continuous cylindrical member, or a strip that is wrapped around the outer circumference of the stent 200, with side edges, which may be parallel or non-parallel to a center longitudinal axis of the prosthetic heart valve, attached to each other so that the outer cuff 270 wraps around the entire circumference of the stent 200.

The stent may be formed from biocompatible materials, including metals and metal alloys such as cobalt chrome (or cobalt chromium) or stainless steel, although in some embodiments the stent may be formed of a shape memory material such as nitinol or the like. The stent is thus configured to collapse upon being crimped to a smaller diameter and/or expand upon being forced open, for example via a balloon within the stent expanding, and the stent will substantially maintain the shape to which it is modified when at rest. The stent may be crimped to collapse in a radial direction and lengthen (to some degree) in the axial direction, reducing its profile at any given cross-section. The stent may also be expanded in the radial direction and foreshortened (to some degree) in the axial direction.

The prosthetic heart valve may be delivered via any suitable transvascular route, for example including transapically or transfemorally. Generally, transapical delivery utilizes a relatively stiff catheter that pierces the apex of the left ventricle through the chest of the patient, inflicting a relatively higher degree of trauma compared to transfemoral delivery. In a transfemoral delivery, a delivery device housing the valve is inserted through the femoral artery and threaded against the flow of blood to the left ventricle. In either method of delivery, the valve may be collapsed and expanded through self-expansion or balloon-expansion, and it will be understood that the present disclosure is intended to address both types of devices. For balloon expansion, the valve may first be collapsed over an expandable balloon while the expandable balloon is deflated. The balloon may be coupled to or disposed within a delivery system, which may transport the valve through the body and heart to reach the aortic valve, with the valve being disposed over the balloon (and, in some circumstance, under an overlying sheath). Upon arrival at or adjacent the aortic valve, a surgeon or operator of the delivery system may align the prosthetic valve as desired within the native valve annulus while the prosthetic valve is collapsed over the balloon. When the desired alignment is achieved, the overlying sheath, if included, may be withdrawn (or advanced) to uncover the prosthetic valve, and the balloon may then be expanded causing the prosthetic valve to expand in the radial direction, with at least a portion of the prosthetic valve foreshortening in the axial direction.

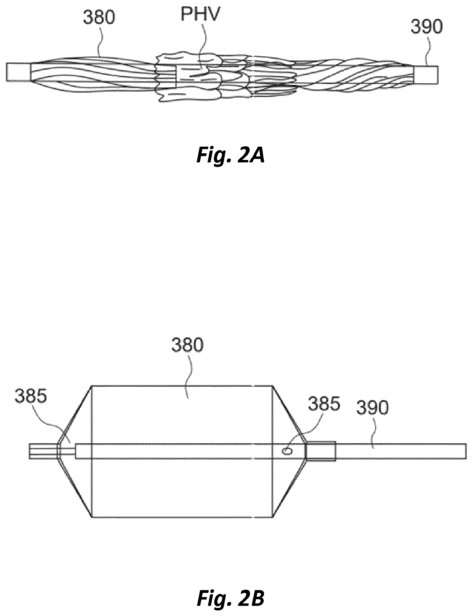

Referring to Fig. 2A, an example of a prosthetic heart valve PHV, which may include a stent similar to stents 100 or 200, is shown crimped over a balloon 380 of a balloon catheter 390 while the balloon 380 is in a deflated condition. It should be understood that other components of the delivery device, such as a handle used for steering and/or deployment, as well as a syringe for inflating the balloon 380, are omitted from Figs. 2A-B. The prosthetic heart valve PHV may be delivered intravascularly, for example through the femoral artery, around the aortic arch, and into the native aortic valve annulus, while in the crimped condition shown in Fig. 2A. Once the desired position is obtained, fluid may be pushed through the balloon catheter 390 to inflate the balloon 380, as shown in Fig. 2B. Fig. 2B omits the prosthetic heart valve PHV, but it should be understood that, as the balloon 380 inflates, it forces the prosthetic heart valve PHV to expand into the native aortic valve annulus (although it should be understood that other heart valves may be replaced using the concepts described herein). In the illustrated example, fluid flows from a syringe or an inflation device (not shown) into the balloon 380 through a lumen within balloon catheter 390 and into one or more ports 385 located internal to the balloon 380. In the particular illustrated example of Fig. 2B, a first port 385 may be one or more apertures in a side wall of the balloon catheter 390, and a second port 385 may be the distal open end of the balloon catheter 390, which may terminate within the interior space of the balloon 380. Though balloon-expandable prosthetic heart valves PHV have been described in some detail, it will be understood that self-expandable prosthetic heart valves PHV may include any suitable combination of the features described above.

As noted above, it would be beneficial to provide a prosthetic heart valve PHV with a smaller diameter that can be easily crimped for loading and delivery. In some embodiments, prosthetic heart valves PHV may be configured and arranged to allow them to be used with relatively smaller delivery devices (e.g., delivery devices that are less than 30 French) in transcatheter valve implants that require a large native anatomical footprint. To resolve issues with conventional devices, the prosthetic heart valves PHV according to the present disclosure aim to axially offset the functional valve assembly from the native anatomy engagement members during delivery, and to allow them to be at least partially overlapped after delivery. Specifically, two components of prosthetic heart valve PHV may include a clearance between them in the loaded condition within a delivery device and the clearance may be eliminated or reduced during deployment. This isolation of the two components may allow for a smaller delivery device as will be appreciated from the embodiments described below.

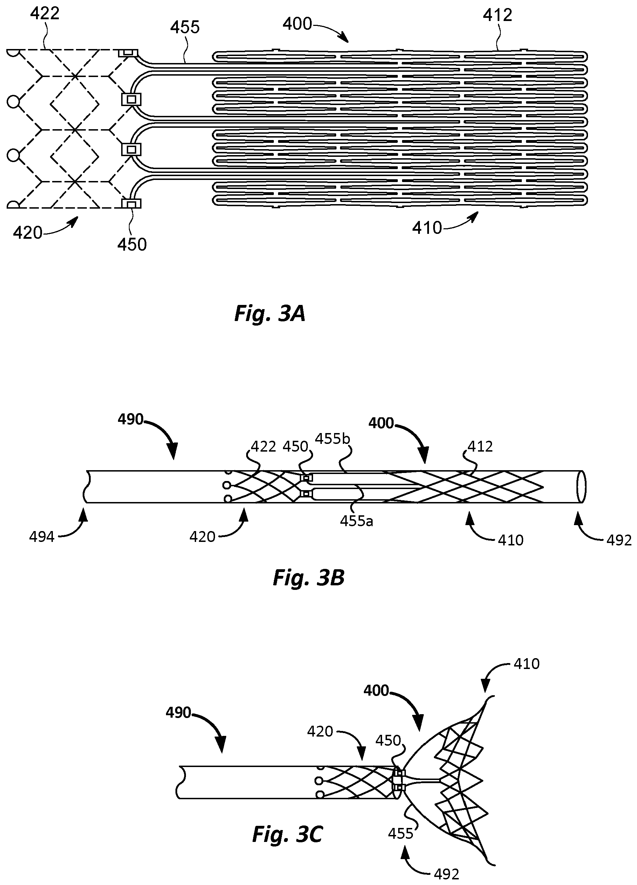

Turning to Figs. 3A-D, a stent assembly 400 is shown that includes an outer stent 410 and an inner stent 420. In this example, the outer stent 410 includes a number of struts 412 sized, configured and arranged to be anchored within the native valve anatomy, while the inner stent 420 includes a number of struts 422 configured to support the functional valve assembly (e.g., the leaflets, cuff, etc.). When deployed (e.g., by releasing the self-expanding stent assembly from a delivery device), these inner and outer stents may partially overlap in an axial direction. Outer and inner stents 410, 420 may be formed of a same material as described above (e.g., nitinol or another shape-memory metal or metal alloy (for self-expanding stents) or steel or cobalt chromium (for balloon-expandable stents). Alternatively, the two stents may be formed of different materials. Additionally, outer and inner stents 410, 420 maybe manufactured together (e.g., by being integrally formed), or may be separately formed and coupled together (e.g., by fusing, welding, gluing or otherwise joining the two stents together). For the sake of clarity, the valve assembly including the cuffs, leaflets and sutures that join them, are not shown. However, it will be understood that any of the preceding configurations of valve assemblies and any number of leaflets may be used in conjunction with stent assembly 400.

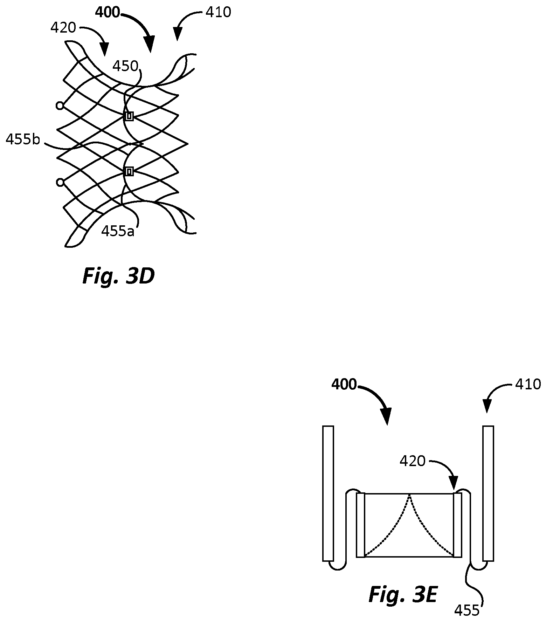

Fig. 3B shows stent assembly 400 disposed within delivery device 490. In this example stent assembly 400 includes an outer stent 410 disposed closer to distal end 492 of delivery device 490, while inner stent 420 is disposed relatively closer to proximal end 494 of delivery device 490. In this example, outer stent 410 will be deployed through distal end 492 and anchored within the native valve anatomy prior to inner stent 420 being released. It will be understood that the inner stent 420 may be disposed closer to distal end 492 than outer stent 410 and that the stents may be oriented in multiple positions within the delivery device depending on which one is to be deployed first. As shown in Fig. 3B, outer stent 410 and inner stent 420 may be joined together via transitionable linking members, and specifically stretchable members 455 that are capable of transitioning between a first condition during delivery (e.g., an elongated condition), and a relaxed, second condition when deployed (e.g., a C-shaped relaxed condition). Stretchable members 455 may be integrated with outer stent 420 *(See,* Fig. 3A) and formed of a shape-set memory material. In some examples, stretchable members 455 are arranged in complementary pairs 455a,455b (*See,* Figs. 3B and 3D) and may be formed of the same and/or a different material than either or both outer stent 420 and inner stent 410 (e.g., nitinol, suture, rubber bands or combinations of materials).

In some examples, stretchable member 455 may be configured as loops that extend beyond any remaining struts of outer stent 410 in the loaded condition (e.g., the stretchable members 455 may extend toward proximal end 494 beyond the rest of the outer stent). In at least some examples, stretchable members 455 may be coupled (e.g., sewn, joined, glued, welded, etc.) to commissure nodes 450 such that the stretchable members 455 urge the commissure nodes 450 and with it the inner stent 420 toward the proximal end of the delivery device during loading. In at least some examples, each commissure node 450 is coupled at a vertex of a designated stretchable member 455, and the number of stretchable members 455 is equal to the number of commissure nodes 450. For example, a prosthetic heart valve PHV may include three leaflets, three commissure nodes and three stretchable members. In at least some examples, in the loaded condition of Fig. 3B, the stretchable members are configured to space the outer stent 410 from inner stent 420 by 0 mm to 60 mm. As stent assembly 400 begins to be deployed and to self-expand upon exiting the delivery catheter (Fig. 3C), outer stent 410 may begin to expand within the native valve anatomy, and stretchable members 455 may begin to foreshorten and form C-shaped members, pulling commissure nodes 450 with them toward the outer stent until the commissure nodes 450 are substantially centered within stent assembly 400 or outer stent 410. In this example, with the stretchable members 455 allowed to return to their C-shaped relaxed condition (Fig. 3D), the inner stent 420 is fully or partially nested within outer stent 410. In at least some examples, commissure nodes 450 are disposed proximal to or distal to the outer stent when the stretchable members 455 are in the collapsed condition, and positioned within, or centered within outer stent 410 (*i.e.,* approximately half-way between the inflow and outflow ends) when the stretchable members 455 are in the relaxed condition. Fig. 3E shows a schematic cross-sectional view of the inner stent 420 being disposed inside the outer stent 410 and connected via stretchable members 455 according to one example.

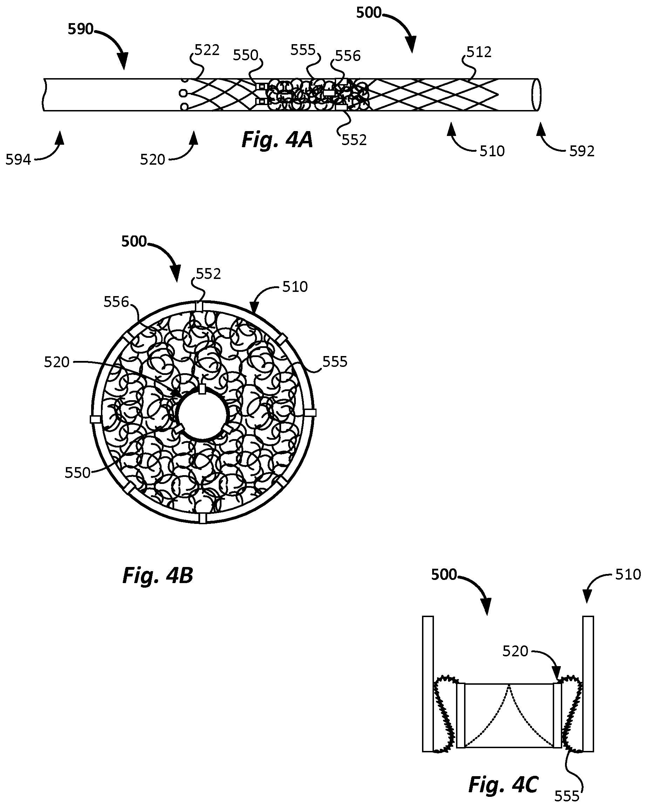

In Figs. 4A-C, another example of a stent assembly 500 is shown that includes an outer stent 510 and an inner stent 520. In this example, the outer stent 510 includes a number of struts 512 sized, configured and arranged to be anchored within the native valve anatomy, while the inner stent 520 includes a number of struts 522 configured to support the functional valve assembly (e.g., the leaflets, cuff, etc.). For the sake of clarity, the valve assembly including the cuffs, leaflets and sutures that join them, are not shown. However, it will be understood that any of the preceding configurations of valve assemblies and any number of leaflets may be used in conjunction with stent assembly 500. Outer and inner stents 510, 520 may be formed of any of the material described above. In this example, outer and inner stents 510, 520 maybe coupled together via an intermediate braided mesh 555.

Fig. 4A shows stent assembly 500 disposed within delivery device 590. In this example stent assembly 500 includes an outer stent 510 disposed closer to distal end 592 of delivery device 590, while inner stent 520 is disposed relatively closer to proximal end 594 of delivery device 590. Outer stent 510 may be deployed through distal end 592 and anchored within the native valve anatomy prior to inner stent 520. As shown in Figs. 4A-B, outer stent 510 and inner stent 520 may be joined together via braided mesh 555 that is capable of transitioning between a first condition during delivery (e.g., a compressed condition), and a second condition when deployed (e.g., a relaxed condition). Braided mesh 555 may be formed of a braided nitinol mesh.

In some examples, braided mesh 555 includes a plurality of wire 556 strands having a predetermined relative orientation between the strands. The metal strands which define two sets of essentially parallel generally helical strands, with the strands of one set having a "hand", i.e., a direction of rotation, opposite that of the other set. The pitch of the wire strands (i.e., the angle defined between the turns of the wire and the axis of the braid) and the pick of the material (i.e., number of wire cross-overs per inch) as well as some other factors, such as the number of wires employed in a braid and their diameter, are important in determining a number of properties of the device. In at least some examples, the number of wires range from 8 to 144. For example, the greater the pick and pitch of the material, and hence the greater the density of the wire strands in the material, the stiffer the device will be for a given wire diameter. Having a greater wire density will also provide the device with a greater wire surface area. Alternatively, two sets of metal strands are used, with one set of strands being oriented at an angle, e.g. generally perpendicular (having a pitch of about 45 degrees), with respect to the other set. As noted above, the pitch and pick of the fabric (or, in the case of a knit material, the pick and the pattern of the material, e.g. Jersey or double knits) may be selected to optimize the desired properties of the resulting medical device.

The wire strands of mesh 555 may be manufactured from so-called shape memory alloys. Such alloys tend to have a temperature induced phase change which will cause the material to have a preferred configuration which can be fixed by heating the material above a certain transition temperature to induce a change in the phase of the material. When the alloy is cooled back down, the alloy will "remember" the shape it was in during the heat treatment and will tend to assume that configuration unless constrained from so doing.

Without any limitation intended, suitable wire strand materials for mesh 555 may be selected from a group consisting of a cobalt-based low thermal expansion alloy referred to in the field as Elgiloy, nickel-based high temperature high-strength "superalloys" commercially available from Haynes International under the trade name HASTELLOY, nickel-based heat treatable alloys sold under the name INCOLOY by International Nickel, and a number of different grades of stainless steel. The important factor in choosing a suitable material for the wire strands is that the wires retain a suitable amount of the deformation induced by a molding surface (as described below) when subjected to a predetermined heat treatment.

In the preferred embodiment, the wire strands 556 of mesh 555 are made from a shape memory alloy, NiTi (known as Nitinol) that is an approximately stoichiometric alloy of nickel and titanium and may also include other minor amounts of other metals to achieve desired properties. Handling requirements and variations of NiTi alloy composition are known in the art, and therefore such alloys need not be discussed in detail here. U.S. Pat. No. 8,779,974 (Amplatz et al.), U.S. Pat. No. 5,067,489 (Lind) and U.S. Pat. No. 4,991,602 (Amplatz et al.), discuss the use of shape memory NiTi alloys in guide wires. Such NiTi alloys are preferred, at least in part, because they are commercially available and more is known about handling such alloys than other known shape memory alloys. NiTi alloys are also very elastic and are said to be "super elastic" or "pseudoelastic". This elasticity allows a device of the invention to return to a preset expanded configuration following deployment.

As shown in Fig. 4A, in the compressed condition, braided mesh 555 may be disposed between outer stent 510 and inner stent 520 and bridge the two components. The braided mesh 555 may be compressed and/or folded in a variety of ways, including being compressed like an accordion. The axial clearance provided by braided mesh 555 may allow the two components to fit within a smaller sized delivery device 590. Turning to Fig. 4B, it can be seen that the braided mesh 555 may be expandable between outer stent 510 and inner stent 520. In at least some examples, braided mesh 555 may be coupled (e.g., sewn, joined, glued, welded, etc.) to outer stent 510 at a plurality of circumferentially spaced outer nodes 552, and to inner stent 520 at a plurality of circumferentially spaced inner nodes 550. In some examples, the inner and outer nodes may represent features on the stents (e.g., vertices of the stent, struts, terminal edges, commissures, or other components). In at least some examples, inner nodes 550 are the commissure features of the inner stent, which are also coupled to the leaflets forming the valve assembly.

In some examples, braided mesh 555 may be configured to extend beyond remaining struts of outer stent 510 in the loaded condition (e.g., the braided mesh 555 may extend toward proximal end 594 beyond the rest of the outer stent). As stent assembly 500 begins to be deployed and to self-expand upon exiting the delivery catheter, outer stent 510 may begin to expand within the native valve anatomy, and braided mesh 555 may begin to radially expand therewith, pulling inner nodes 550 with them until the inner nodes 550 are substantially centered within stent assembly 500. In this example, with the braided mesh 555 allowed to return to its relaxed condition, the inner stent 520 is fully or partially nested within outer stent 510. In at least some examples, inner nodes 550 are disposed proximal to the outer stent when the braided mesh 555 is in the collapsed condition, and centered within outer stent *510 (i.e.,* approximately half-way between the inflow and outflow ends) when the braided mesh 555 is in the relaxed condition. Fig. 4C shows a schematic cross-sectional view of the inner stent 510 being disposed inside the outer stent 510 and connected via braided mesh 555 according to one example.

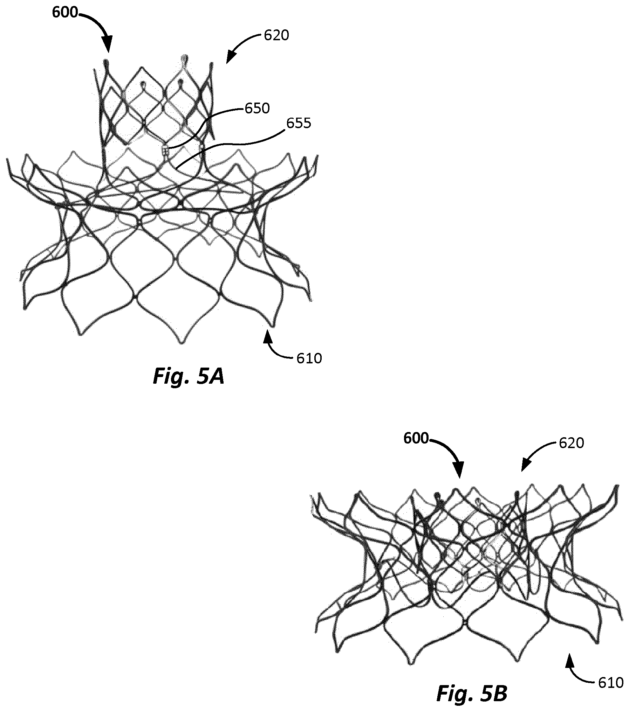

In another variation, shown in Figs. 5A-B, a stent assembly 600 may include an outer stent 610 and an inner stent 620, the two stents being joined together via integrated linking struts 655. Linking struts 655 may function in a manner similar to stretchable members 455 described above by axially distancing the outer stent from the inner stent during loading and delivery, and bringing the two stents together during deployment. Fig. 5A shows the two stents as they would appear after the outer stent 610 has been placed in the native valve anatomy, and while inner stent 620 is being pulled within outer stent 610 via linking struts 655.

In at least some examples, integrated linking struts 655 may be joined to cells or vertices of cells of outer stent 610 and to commissure nodes 650 of inner stent 620. In the example shown, two linking struts 655 are coupled to each commissure node 650 to form an inverted V-shaped spacer from commissure node 650, and the linking struts 655 are of a sufficient length so as to allow the inner and outer stents to be axially offset during loading and delivery (Fig. 5A), and to pull the two components together so that they are nested after deployment (Fig. 5B). In at least some examples, the inverted V-shaped spacers are substantially axially-oriented (e.g., the axial component is greater than the radial component) in the compressed delivery condition, and substantially radially-oriented (e.g., the radial component is greater than the axial component) in the expanded condition when implanted.

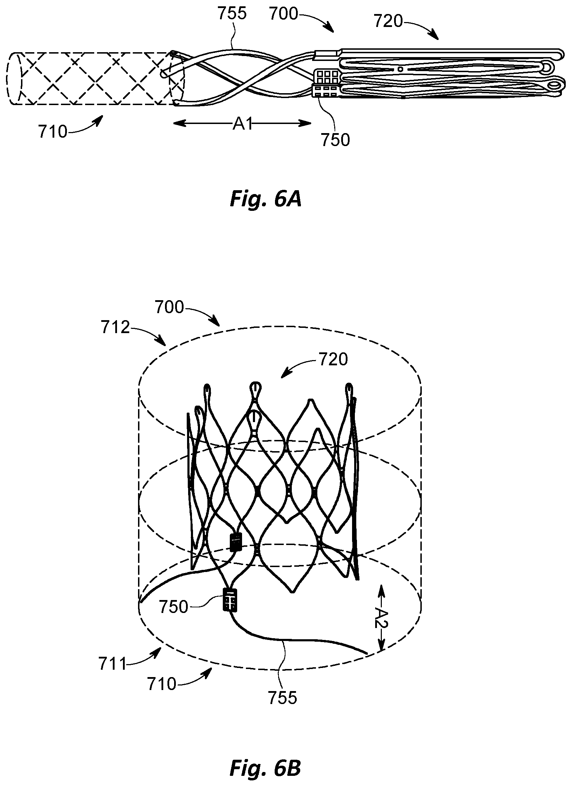

In another variation, shown in FIG. 6A-B, a stent assembly 700 may include an outer stent 710 and an inner stent 720, the two stents being joined together via spiral linking struts 755. In this example, a dashed generic tube appears where outer stent 710 would be placed, but it will be understood that outer stent 710 may take any of the configurations described above and may have struts defining cells (e.g., diamond-shaped) as would be expected from laser cutting a stent from a tube.

In this example, three spiral linking struts 755 couple the inner and outer stents 720,710 together and the three spiral linking struts 755 may transition between a compressed condition and a relaxed condition. Spiral linking struts 755 may be formed of a shape-memory material (e.g., nitinol or nitinol braid). It will be understood that more or less spiral linking struts 755 may be used (e.g., one, two, three, four, five, six, seven, eight or more spiral linking struts). As shown, each spiral linking strut 755 may be joined to a respective commissure node 750, but it will be understood that each commissure node 750 may be coupled to two or more spiral linking struts 755. In some examples, each spiral linking strut 755 may form or, be oriented, along a helical pattern, and the three struts 755 each spiral in the same orientation (e.g., clockwise or counter-clockwise). Additionally, each spiral linking strut 755 may form one full revolution or less (e.g., each spiral linking strut 755 may complete a rotation of 30, 60, 90, 120, 150, 180, 210, 240, 270, 300 or 330 degrees) in the compressed condition (Fig. 6A). In this compressed condition of the linking struts 755, the outer stent 710 may be spaced from the inner stent 720 by a first axial distance A1 that is approximately 5 and 70 mm.

Fig. 6B shows the two stents 710,720 as they might appear after the outer stent 710 has been placed in the native valve anatomy and inner stent 720 is brought within outer stent 710 via spiral linking struts 755. In this expanded configuration of the stent assembly 700, spiral linking struts 755 span a second axial distance A2 that is smaller than first axial distance A1. In some examples, the struts 755 in their relaxed configuration extend substantially radially outward so that the second axial distance A2 is minimal or close to zero, that is the struts 755 extend radially outward with no axial component to the extension. In some examples, the struts 755 are more tightly wound in a compressed condition than in a relaxed condition (e.g., they form more turns or portions of at turn when compressed than in a relaxed condition). Although the spiral linking struts 755 are shown as being attached to the proximal end 711 of outer stent 710, it will be understood that they could also be joined or coupled to the distal end 712 or at any portion therebetween.

In use, a prosthetic heart valve may be manufactured according to any of the manners and configurations described above with an intermediate linking member (e.g., stretchable members, loops, braided mesh, linking struts) that couples and inner stent to an outer stent. The prosthetic heart valve may be loaded within a delivery device such that the inner and outer stents are axially offset (e.g., unnested) from one another by the intermediate linking member. In the partially or fully expanded state of prosthetic heart valve PHV, the outer stent may begin to expand within the native valve anatomy, for example, through self-expansion. As more of the stent assembly is deployed, the intermediate linking member may begin to transition from the compressed condition to a relaxed condition where the linking member radially expands, foreshortens, straightens and/or otherwise changes orientation and/or shape, to allow the inner stent to be brought within the outer stent so that the two stents are partially or fully nested. Thus, a relatively large prosthetic heart valve may be delivered with a smaller profile delivery device. These proposed configurations may be particularly be advantageous in the right heart where the pressures are low enough that high ventricular pressures may not interfere with the stability of the inner/outer stent designed proposed in this disclosure. Additionally, methods are contemplated that would allow for a progressive retrieval via the trans-femoral access.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. A prosthetic heart valve system, comprising:
an outer stent (610, 710);
an inner stent (620, 720) coupled to the outer stent and including a plurality of commissure nodes (650, 750), the inner stent supporting a valve assembly including a cuff and a plurality of leaflets; and
multiple linking members (655, 755) coupling the outer stent to the inner stent, each of the linking members (655, 755) being transitionable between a collapsed condition and a relaxed condition, the inner stent (620, 720) being axially spaced away from the outer stent (610, 710) when the linking members are in the collapsed condition, and the inner stent being at least partially nested within the outer stent when the linking members are in the relaxed condition, wherein each of the plurality of commissure nodes (650, 750) is coupled to a corresponding one of the linking members.

2. The prosthetic heart valve system of claim 1, wherein the plurality of commissure nodes (650, 750) is disposed proximal to the outer stent (610, 710) when the linking members (655, 755) are in the collapsed condition, and the plurality of commissure nodes (650, 750) is nested within the outer stent when the linking members (655, 755) are in the relaxed condition.

3. The prosthetic heart valve system of claim 1, wherein each of the linking members (655, 755) is C-shaped in the relaxed condition.

4. The prosthetic heart valve system of claim 1, wherein each of the linking members (655, 755) extends past a remainder of the outer stent (610, 710) in the collapsed condition.

5. The prosthetic heart valve system of claim 1, wherein the inner stent (620, 720) is fully nested within the outer stent (610, 710) when the linking members (655, 755) are in the relaxed condition.

6. The prosthetic heart valve system of claim 1, wherein each of the linking members includes a spiral linking strut (755) having a helical configuration.

7. The prosthetic heart valve system of claim 6, wherein each of the plurality of commissure nodes (750) is coupled to one spiral linking strut (755).

8. The prosthetic heart valve system of claim 7, wherein the inner stent (720) includes three commissure nodes (750) and three spiral linking struts (755).

9. The prosthetic heart valve system of claim 6, wherein each spiral linking strut (755) is integrally formed with the inner stent (720).

10. The prosthetic heart valve system of claim 6, wherein each spiral linking strut (755) is more tightly wound in the collapsed condition than in the relaxed condition.

11. The prosthetic heart valve system of claim 1, wherein each of the plurality of commissure nodes (650) is coupled to two linking members (655).

12. The prosthetic heart valve system of claim 11, wherein the two linking members (655) form a V-shaped spacer, and each V-shaped spacer is substantially axially-oriented in the collapsed condition, and substantially radially-oriented in the relaxed condition.

13. The prosthetic heart valve system of claim 1, wherein the inner stent (620, 720) has no axial overlap with the outer stent (610, 710) when the linking members (655, 755) are in the collapsed condition.

## Patentansprüche

1. Ein Herzklappenprothese-System, aufweisend:
einen äußeren Stent (610, 710);
einen inneren Stent (620, 720), der mit dem äußeren Stent verbunden ist und mehrere Kommissurknoten (650, 750) aufweist, wobei der innere Stent eine Klappenanordnung mit einer Manschette und mehreren Klappensegeln stützt; und
mehrere Verbindungselemente (655, 755), die den äußeren Stent mit dem inneren Stent verbinden, wobei jedes der Verbindungselemente (655, 755) zwischen einem kollabierten Zustand und einem entspannten Zustand übergangsfähig ist, wobei der innere Stent (620, 720) axial vom äußeren Stent (610, 710) beabstandet ist, wenn sich die Verbindungselemente im kollabierten Zustand befinden, und wobei der innere Stent zumindest teilweise innerhalb des äußeren Stents angeordnet ist, wenn sich die Verbindungselemente im entspannten Zustand befinden, wobei jeder der mehreren Kommissurknoten (650, 750) mit einem entsprechenden der Verbindungselemente verbunden ist.

2. Das Herzklappenprothese-System nach Anspruch 1, wobei die mehreren Kommissurknoten (650, 750) proximal zum äußeren Stent (610, 710) angeordnet sind, wenn sich die Verbindungselemente (655, 755) im kollabierten Zustand befinden, und die mehreren Kommissurknoten (650, 750) innerhalb des äußeren Stents angeordnet sind, wenn sich die Verbindungselemente (655, 755) im entspannten Zustand befinden.

3. Das Herzklappenprothese-System nach Anspruch 1, wobei jedes der Verbindungselemente (655, 755) im entspannten Zustand C-förmig ist.

4. Das Herzklappenprothese-System nach Anspruch 1, wobei jedes der Verbindungselemente (655, 755) im kollabierten Zustand über einen Teil des äußeren Stents (610, 710) hinausragt.

5. Das Herzklappenprothese-System nach Anspruch 1, wobei der innere Stent (620, 720) vollständig innerhalb des äußeren Stents (610, 710) angeordnet ist, wenn sich die Verbindungselemente (655, 755) im entspannten Zustand befinden.

6. Das Herzklappenprothese-System nach Anspruch 1, wobei jedes der Verbindungselemente eine spiralförmige Verbindungsstrebe (755) mit einer schraubenförmigen Konfiguration aufweist.

7. Das Herzklappenprothese-System nach Anspruch 6, wobei jeder der mehreren Kommissurknoten (750) mit einer spiralförmigen Verbindungsstrebe (755) verbunden ist.

8. Das Herzklappenprothese-System nach Anspruch 7, wobei der innere Stent (720) drei Kommissurknoten (750) und drei spiralförmige Verbindungsstreben (755) aufweist.

9. Das Herzklappenprothese-System nach Anspruch 6, wobei jede spiralförmige Verbindungsstrebe (755) integral mit dem inneren Stent (720) ausgebildet ist.

10. Das Herzklappenprothese-System nach Anspruch 6, wobei jede spiralförmige Verbindungsstrebe (755) im kollabierten Zustand enger gewunden ist als im entspannten Zustand.

11. Das Herzklappenprothese-System nach Anspruch 1, wobei jeder der mehreren Kommissurknoten (650) mit zwei Verbindungselementen (655) verbunden ist.

12. Das Herzklappenprothese-System nach Anspruch 11, wobei die beiden Verbindungselemente (655) einen V-förmigen Abstandhalter bilden und jeder V-förmige Abstandhalter im kollabierten Zustand im Wesentlichen axial ausgerichtet ist und im entspannten Zustand im Wesentlichen radial ausgerichtet ist.

13. Das Herzklappenprothese-System nach Anspruch 1, wobei der innere Stent (620, 720) keine axiale Überlappung mit dem äußeren Stent (610, 710) aufweist, wenn sich die Verbindungselemente (655, 755) im kollabierten Zustand befinden.

## Revendications

1. Un système de valve cardiaque prothétique, comprenant :
un stent externe (610, 710) ;
un stent interne (620, 720) couplé au stent externe et comprenant une pluralité de nœuds commissuraux (650, 750), le stent interne supportant un ensemble de valve comprenant un manchon et une pluralité de feuillets ; et
plusieurs éléments de liaison (655, 755) reliant le stent externe au stent interne, chacun des éléments de liaison (655, 755) pouvant passer d'un état replié à un état détendu, le stent interne (620, 720) étant espacé axialement du stent externe (610, 710) lorsque les éléments de liaison sont dans la position repliée, et le stent interne étant au moins partiellement emboîté dans le stent externe lorsque les éléments de liaison sont dans la position détendue, dans lequel chacun de la pluralité de nœuds de commissure (650, 750) est couplé à un élément de liaison correspondant parmi les éléments de liaison.

2. Le système de valve cardiaque prothétique selon la revendication 1, dans lequel la pluralité de nœuds commissuraux (650, 750) est disposée à proximité du stent externe (610, 710) lorsque les éléments de liaison (655, 755) sont dans l'état replié, et la pluralité de nœuds de commissure (650, 750) est nichée à l'intérieur du stent externe lorsque les éléments de liaison (655, 755) sont dans l'état détendu.

3. Le système de valve cardiaque prothétique selon la revendication 1, dans lequel chacun des éléments de liaison (655, 755) est en forme de C dans l'état détendu.

4. Le système de valve cardiaque prothétique selon la revendication 1, dans lequel chacun des éléments de liaison (655, 755) s'étend au-delà d'un reste du stent externe (610, 710) dans l'état replié.

5. Le système de valve cardiaque prothétique selon la revendication 1, dans lequel le stent interne (620, 720) est entièrement emboîté dans le stent externe (610, 710) lorsque les éléments de liaison (655, 755) sont à l'état détendu.

6. Le système de valve cardiaque prothétique selon la revendication 1, dans lequel chacun des éléments de liaison comprend une entretoise de liaison en spirale (755) ayant une configuration hélicoïdale.

7. Le système de valve cardiaque prothétique selon la revendication 6, dans lequel chacun des multiples nœuds de commissure (750) est couplé à une entretoise de liaison en spirale (755).

8. Le système de valve cardiaque prothétique selon la revendication 7, dans lequel le stent interne (720) comprend trois nœuds de commissure (750) et trois entretoises de liaison en spirale (755).

9. Le système de valve cardiaque prothétique selon la revendication 6, dans lequel chaque entretoise de liaison en spirale (755) est formée d'un seul tenant avec le stent interne (720).

10. Le système de valve cardiaque prothétique selon la revendication 6, dans lequel chaque entretoise de liaison en spirale (755) est enroulée plus étroitement à l'état replié qu'à l'état détendu.

11. Le système de valve cardiaque prothétique selon la revendication 1, dans lequel chacun des multiples nœuds de commissure (650) est couplé à deux éléments de liaison (655).

12. Le système de valve cardiaque prothétique selon la revendication 11, dans lequel les deux éléments de liaison (655) forment une entretoise en forme de V, et chaque entretoise en forme de V est orientée sensiblement axialement dans l'état replié, et orientée sensiblement radialement dans l'état détendu.

13. Le système de valve cardiaque prothétique selon la revendication 1, dans lequel le stent interne (620, 720) ne présente aucun chevauchement axial avec le stent externe (610, 710) lorsque les éléments de liaison (655, 755) sont à l'état replié.
